# NEW EUROPEAN PATENT SPECIFICATION

(11) **EP 1 674 132 B2**
(45) Date of publication and mention of the opposition decision: **13.04.2011**
(45) Mention of the grant of the patent: 13.02.2008
(21) Application number: 04029772.3
(22) Date of filing: 16.12.2004
(51) Int. Cl.: A61Q 5/02, A61Q 5/12, A61Q 19/10, A61K 8/365, A61K 8/46, A61K 8/60, A61K 8/44, C11D 1/94, C11D 3/20

(54) **Mild cleansing composition**
Milde Reinigungszusammensetzung
Composition de nettoyage douce

(43) Date of publication of application: 28.06.2006
(73) Proprietor: KPSS-Kao Professional Salon Services GmbH, 64297 Darmstadt (DE)
(72) Inventor: Molenda, Michael, 60598 Frankfurt (DE)

(56) References cited:
- EP-A2- 0 733 355
- DE-A1- 19 613 567
- DE-A1- 19 964 155
- DE-C1- 19 729 080
- GB-A- 2 303 637
- US-A- 4 009 256
- US-A- 5 683 685
- US-A1- 2004 101 504

## Description

The present invention is related to cleansing composition with improved mildness for skin and hair, especially for hair.

Mildness of a cleansing composition is a very important issue due to its frequent use. Improvements in this area have been disclosed in many publications However, majority of the disclosures is concerned with cleansing compositions around neutral pH values, more specifically between pH 5.0 and 7.0.

Recently, acidic shampoos have been developed having pH values below pH 5.0, more specifically below 4.5 and shown to be superior in the hair conditioning performance and especially shine improvement, natural feeling and manageability of hair (EP 10 16 401 A1). There has been very little information available on the mildness of such cleansing compositions.

Cleansing and/or conditioning hair cosmetic compositions are disclosed in GB 2 303 637 A, US 4,009,256 and US 5,683, 685 based on surfactants and conditioning ingredients. None of these discloses a composition falling under the claims of the present invention.

US 2004/0101504 A1 discloses mild antibacterial liquid dish washing detergent composition.

However, problems have been encountered in mildness of cleansing compositions having pH especially below 4.5. In the use of such cleansing compositions, itchiness, redness and dry feeling of skin and scalp have been reported.

Recently it has surprisingly been found out that a cleansing composition having pH below 4.5 and comprising at least one anionic surfactant, at least one nonionic surfactant and at least one amphoteric or zwitterionic surfactant at a certain weight ratio is especially mild to skin and scalp. Compositions are suitable for use as skin cleansing and as well as hair cleansing and conditioning compositions - shampoo. Therefore, throughout the disclosure of the present invention the term cleansing composition is used and meant skin and hair cleansing compositions.

Accordingly, the present invention is on a cleansing composition comprising at least one anionic surfactant, at least one nonionic surfactant and at least one amphoteric or zwitterionic surfactant at a weight ratio between 10/2.5/1 and 10/5/2 at least one hydroxy carboxylic acid and/or dicarboxylic acid selected from lactic and malic acids and having a pH below 4.5.

The preferred weight ratio of at least one anionic surfactant, at least one nonionic surfactant and at least one amphoteric or zwitterionic surfactant is in the range of 10/3/1 to 10/4/2 and most preferably the ratio is 10/3.8/1.4.

The pH of the cleansing compositions is preferably 2 to below 4.5, more preferably 2.5 to 4.0, most preferably 2.9 to 3,8.

In principal the pH of the compositions can be adjusted with any organic and/or inorganic acids or their mixture. Some of them to mention are phosphoric acid, hydrochloric acid as the inorganic ones and to the organic acids the well known citric acid. However, the best hair conditioning effect is observed in terms of conditioning especially of hair and mildness with the carboxylic acids and especially those of with hydroxycarboxylic acids and/or dicarboxylic acids. In those cases where selected hydroxycarboxylic acid and/or dicarboxylic acid concentration is not enough to reach the selected pH, other organic and inorganic acids can as well be used to adjust the pH to the required value. The hydroxycarboxylic acids useful therefore are glycolic acid, hydroxyacrylic acid, glyceric acid, and tartaric acid and of the dicarboxylic acids are malonic acid, succinic acid, glutaric acid, adipic acid, maleic acid, fumaric acid and phtalic acid.

The most preferred hydroxycarboxylic acid and/or dicarboxylic acid is the malic acid.

Total hydroxycarboxylic acid and/or dicarboxylic acid concentration in the composition of the present invention varies in the range form 0.1 to 5% by weight, preferably 0.25 to 3% by weight, more preferably 0.5 to 3% by weight and most preferably 0.75 to 3% by weight. In a preferred embodiment of the invention, the compositions of the present invention comprise at least 0.5% malic acid.

Cleansing compositions of the present invention comprises surfactants at a total concentration of 1 to 50%, preferably 5 to 40% and more preferably 5 to 30%, and most preferably 5 to 25% by weight, calculated to the total composition.

Anionic surfactants suitable within the scope of the invention are preferably present in an amount from 1 to 30%, preferably 2 to 20% and most preferably 2 - 15%, and most preferably 3 to 15% by weight, calculated to the total composition.

These are anionic surfactants of the sulfate, sulfonate, carboxylate and alkyl phosphate type, especially, of course, those customarily used in shampoo compositions, for example, the known C₁₀-C₁₈-alkyl sulfates, and in particular the respective ether sulfates, for example, C₁₂-C₁₄-alkyl ether sulfate, lauryl ether sulfate, especially with 1 to 4 ethylene oxide groups in the molecule. Other anionic surfactants can as well be included into the cleansing compositions of the present invention such as monoglyceride (ether) sulfates, fatty acid amide sulfates obtained by ethoxylation and subsequent sulfatation of fatty acid alkanolamides, and the alkali salts thereof, as well as the salts of long-chain mono- and dialkyl phosphates constituting mild, skin-compatible detergents.

Additional anionic surfactants useful within the scope of the invention are alkali salts of sulfosuccinic acid semiesters, for example, the disodium salt of monooctyl sulfosuccinate and alkali salts of long-chain monoalkyl ethoxysulfosuccinates.

Suitable surfactants of the carboxylate type are alkyl polyether carboxylic acids and the salts thereof of the formula

R₁- (C₂H₄O)ₙ- O- CH₂COOX,

wherein R₁ is a C₈-C₂₀-alkyl group, preferably a C₁₂-C₁₄-alkyl group, n is a number from 1 to 20, preferably 2 to 17, and X is H or preferably a cation of the group sodium, potassium, magnesium and ammonium, which can optionally be hydroxyalkyl-substituted.

Further suitable anionic surfactants are also C₈-C₂₂-acyl aminocarboxylic acids or the water-soluble salts thereof. Especially preferred is N-lauroyl glutamate, in particular as sodium salt, as well as, for example, N-lauroyl sarcosinate, N-C₁₂-C₁₈-acyl asparaginic acid, N-myristoyl sarcosinate, N-oleoyl sarcosinate, N-lauroyl methylalanine, N-lauroyl lysine and N-lauroyl aminopropyl glycine, preferably in form of the water-soluble alkali or ammonium, in particular the sodium salts thereof, preferably in admixture with the above-named anionic surfactants.

It is also possible to use mixtures of several anionic surfactants, for example an ether sulfate and a polyether carboxylic acid or C₈-C₂₂-acyl aminocarboxylic acids. The most preferred anionic surfactant is ethoxlated fatty alcohol sulfates and among them the most preferred is sodium lauryl ether sulfate known with its trade name for example Texapon from the company Henkel.

The cleansing compositions of the present invention comprise at least one nonionic surfactant at a concentration of 1 to 15%, preferably 1 to 10% and more preferably 1 to 7.5% and most preferably 2 to 5 % by weight calculated to the total composition. When determining the concentration of nonionic surfactant as well known by the skilled worker the weight ratio with the anionic surfactant must be observed.

These are described in Schrader, I.c., on pages 600-601 and pp. 694-695. Especially suited are alkyl polyglucosides of the general formula

R₂-O-(R₃O)ₙ-Zₓ,

wherein R₂ is an alkyl group with 8 to 18 carbon atoms, R₃ is an ethylene or propylene group, Z is a saccharide group with 5 to 6 carbon atoms, n is a number from 0 to 10 and x is a number between 1 and 5. The nonionic surfactants are the most preferred ones according to the preferred embodiment of the invention. Among those the most preferred is Coco glucoside known with the trade name Plantacare 818 UP.

Further nonionic surfactant components are, for example, long-chain fatty acid mono- and dialkanolamides, such as coco fatty acid monoethanolamide and myristic fatty acid monoethanolamide, which may also be used as foam enhancers.

Further additionally useful nonionic surfactants are, for example, the various sorbitan esters, such as polyethylene glycol sorbitan stearic acid ester, fatty acid polyglycol esters or poly-condensates of ethyleneoxide and propyleneoxide, as they are on the market, for example, under the trade name "Pluronics^{R}", as well as fatty alcohol ethoxylates.

Further nonionic surfactants useful in the compositions according to invention are C₁₀-C₂₂-fatty alcohol ethoxylates at a concentration of 0.5 to 10%, preferably 0.5 to 5% by weight, calculated to total composition. Especially suited are C₁₀-C₂₂-fatty alcohol ethers, the alkyl polyglycol ethers known by the generic terms "Laureth", "Myristeth", "Oleth", "Ceteth", "Deceth", "Steareth" and "Ceteareth" according to the CTFA nomenclature, including addition of the number of ethylene oxide molecules, e.g., "Laureth-16":

The average degree of ethoxylation thereby ranges between 2.5 and 25, preferably 10 and 20.

As further surfactant component, the compositions according to the invention comprise at least one amphoteric or zwitterionic surfactants, for example in an amount from 0.5 % to 15 %, preferably from 1 % to 10 %, and more preferably from 1 to 7.5%, most preferably from 1 to 5% by weight, calculated to the total composition. It has especially been found out that addition of zwitterionic or amphoteric surfactants enhances foam feeling in terms of creaminess, foam volume. Here again when determining the concentration of amphoteric and/or zwitterionic surfactants as well known by the skilled worker, the weight ratio with the anionic surfactants must be observed.

Useful as such are in particular the various known betaines such as alkyl betaines, fatty acid amidoalkyl betaines and sulfobetaines, for example, lauryl hydroxysulfobetaine; long-chain alkyl amino acids, such as cocoaminoacetate, cocoaminopropionate and sodium cocoamphopropionate and -acetate have also proven suitable.

In detail, it is possible to use betaines of the structure 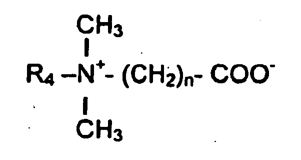 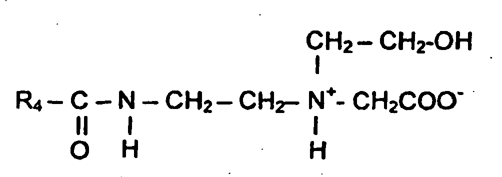 wherein R₄ is a C₈-C₁₈-alkyl group and n is 1 to 3;
sulfobetaines of the structure 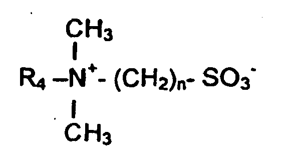 wherein R₄ and n are same as above;
and amidoalkyl betaines of the structure 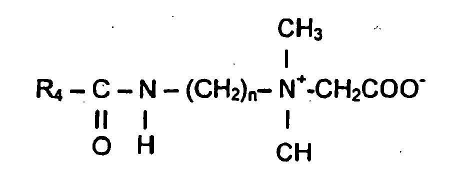 wherein R₄ and n are same as above.

The preferred amphoteric and/or zwitterionic surfactants are alkyl amidoalkyl betaines and among those cocamidopropyl betaine.is the most preferred one.

According to another preferred embodiment of the invention, the compositions of the present invention comprise conditioning agents. Conditioning agents can be selected from oily substances, non ionic substances, cationic amphiphilic ingredients, cationic polymers or their mixtures. Without making any limitation cationic conditioning compounds are especially preferred in the case of compositions for hair. Again without making any limitation nonionic conditioners either oily or water soluble are found to be more suitable for skin cleansing compositions.

Oily substances are selected from such as silicone oils, either volatile or nonvolatile, natural and synthetic oils. Among silicone oils those can be added to the compositions include dimethicone, dimethiconol, polydimethylsiloxane, DC fluid ranges from Dow Corning, natural oils such as olive oil, almond oil, avocado oil, weizenkeim oil, ricinus oil and the synthetic oils, such as mineral oil, isopropyl myristate, palmitate, stearate and isostearate, oleyl oleate, isocetyl stearate, hexyl laurate, dibutyl adipate, dioctyl adipate, myristyl myristate and oleyl erucate.

Non ionic conditioning agents may be polyols such as glycerin, glycol and derivatives, polyethyleneglycoles known with trade names Carbowax PEG from Union Carbide and Polyox WSR range from Amerchol, polyglycerin, polyethyleneglycol mono or di fatty acid esters having general formula

R₅ CO (O CH₂ CH₂)ₙ OH

or

R₅ CO (O CH₂ CH₂)ₙ O OC R₆

where R₅ and R₆ are independent from each other saturated, unsaturated or branched or non-branched alkyl chain with 7 to 21 C atoms and n is typically 2 - 100.

Suitable cationic polymers as conditioning agents are those of best known with their CTFA category name Polyquaternium. Typical examples of those Polyquaternium 6, Polyquaternium 7, Polyquaternium 10, Polyquaternium 11, Polyquaternium 16, Polyquaternium 22 and Polyquaternium 28.

As well those polymers known with their CTFA category name Quaternium are suitable. Those are for example Quaternium-8, Quaternium-14, Quaternium-15, Quatemium-18, Quatemium-22, Quatemium-24, Quatemium-26, Quatemium-27, Quatemium-30, Quatemium-33, Quatemium-53, Quaternium-60, Quatemium-61, Quatemium-72, Quatemium-78, Quaternium-80, Quaternium-81, Quaternium-81, Quaternium-82, Quaternium-83 and Quaternium-84,

It has further been found out that especially those of cationic cellulose type polymers known as Polymer JR type from Amerchol such as Polyquaternium 10 or cationic guar gum known with trade name Jaguar from Rhône-Poulenc and chemically for example Guar hydroxypropyl trimonium chloride, are preferred ones. Furthermore, chitosan and chitin can also be included in the compositions as cationic natural polymers. In this context reference is also made to the cationic polymers disclosed in DE 25 21 960, 28 11 010, 30 44 738 and 32 17 059, as well as to the products described in EP-A 337 354 on pages 3 to 7. It is also possible to use mixtures of various cationic polymers.

The most preferred cationic polymers are those of cationic cellulose derivatives, cationic guar gum derivatives, polyquaternium 6 and polyquaternium 7.

The cationic polymers also include the quaternized products of graft polymers from organopolysiloxanes and polyethyl oxazolines described in EP-A 524 612 and EP-A 640 643.

Cleansing compositions of the present invention can comprise one or more cationic surfactant(s) as conditioner presented with the general formula 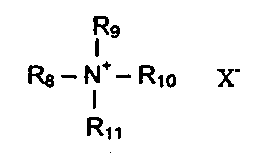 where R₈ is a saturated or unsaturated, branched or non-branched alkyl chain with 8-22 C atoms or

R₁₂ CO NH (CH₂)ₙ

where R₁₂ is saturated or unsaturated, branched or non-branched alkyl chain with 7-21 C atoms and n has value of 1 - 4, or

R₁₃ CO O (CH₂)ₙ

where R₁₃ is saturated or unsaturated, branched or non-branched alkyl chain with 7-21 C atoms and n has value of 1 - 4, and
R₉ is hydrogen or unsaturated or saturated, branched or non-branched alkyl chain with 1 - 4 C atoms or

R₁₂ CO NH (CH₂)ₙ

or

R₁₃ CO O (CH₂)ₙ

where R₁₂, R₁₃ and n are same as above.

R₁₀ and R₁₁ are hydrogen or lower alkyl chain with 1 to 4 carbon atoms, and X is anion such as chloride, bromide, methosulfate.

Typical examples of those ingredients are cetyl trimethly ammonium chloride, stear trimonium chloride, behentrimonium chloride, stearamidopropyl trimonium chloride, dioleoylethyl dimethyl ammonium methosulfate, dioleoylethyl hydroxyethylmonium methosulfate.

The compositions according to the invention may also comprise further conditioning substances such as protein hydrolyzates and polypeptides, e.g., keratin hydrolyzates, collagen hydrolyzates of the type "Nutrilan^{R}" or elastin hydrolyzates, as well as also in particular plant protein hydrolyzates, optionally, cationized protein hydrolyzates, e.g., "Gluadin^{R}".

Typical concentration range for any of those conditioners of cationic polymers, silicon oil and derivatives and cationic surfactants can be 0.01 - 5% by weight, preferably 0.01 - 3.5% by weight, more preferably 0.05 - 2.5% and most preferably 0.1 - 1.5% by weight calculated to the total composition.

Cleansing compositions may comprise organic solvents such as ethanol, propanol, isopropanol, benzyl alcohol, benzyloxyethanol, ethoxydiglycol, alkylene carbonates such as ethylene carbonate and propylene carbonate, phenoxyethanol, butanol, isobutanol, cyclohexane, cyclohexanol, hexyleneglycol, ethylenecarbonate, propyleneglycol, poypropyleneglycols, ethyleneglycol monoethylether, ethylene glycol monobutyl ether, ethylene glycol monophenyl ether, 1-phenylethylalcohol, 2-phenylethylalcohol, o-methoxyphenol. The most preferred ones are benzylalcohol, benzyloxyethanol and polypropylene glycols. Concentration of organic solvents in the cleasning compositions should not exceed 5% by weight, preferably in the range of 0.1 to 3%, more preferably 0.5 to 2.5% by weight calculated to total composition.

Solubilizers may be added to the compositions especially when oily substances are chosen as conditioning agents and fragrance oils with highly lipophilic properties. Typical solubilizers may be hydrogenated castor oil known with the trade mark Cremophor RH series from BASF. It should be noted that as well the surfactant mixture can be a good solubilizer for fragrance oils. Typical concentration of the solubilizers can be in the range of 0.01 - 2% by weight, preferably 0.1 - 1 % by weight, calculated to total composition.

Cleansing compositions may be transparent as well as pearly. Transparency of the composition is judged by naked eye in a transparent shampoo bottle with a thickness not more than 5 cm. In the case a transparent appearance is wished, the following ingredients are not essential. Pearl-shiny appearance is achieved with those dispersed in liquid cleansing compositions in crystalline form, i.e. so called pearl-shine or pearlizing agents. The preferred once are PEG-3 distearate and ethylene glycol distearate. The concentration of those can typically be from 0.1 to 3%, preferably 0.5 to 2% by weight, calculated to the total composition. These compounds are preferably added to the compositions in admixture with anionic, nonionic and/or amphoteric surfactants. Such kind of mixtures is available commercially.

The viscosity of the cleansing compositions according to the invention is in the range of 500 and 20,000 mPa.s at 20°C, preferably 1,000 to 10,000, in particular 1,500 to 8,000 mPa.s at 20°C, measured with Höppler viscosimeter at a shear rate of 10 sec⁻¹. Viscosity of compositions can be adjusted with known viscosity enhancers. The preferred ones are glyceryl laurate, PEG-55 propyleneglycol oleate and PEG-18 glyceryl oleate/cocoate known with the trade names Antil^{R} 141 and 171, respectively and PEG-160 sorbitan triisostearate known with a trade name Rheodol^{R}. It should be noted that in the case that a composition are delivered in the form of a foam from a pump-foamer and/or aerosol can, those compositions should not be thickened and have a viscosity value not more than 500 mPa.s, more preferably 250 mPa.s measured as mentioned above at room temperature. In the case that a cleansing composition in an aerosol form is preferred, propellants such as dimethylether, propane, butane, isobutene must be included as a presurizer.

The compositions of the present invention may comprise active ingredients selected from UV filters, moisturisers, sequestering agents, and natural ingredients.

The moisturizing agents are selected from panthenol, polyols, such as glycerol, polyethylene glycols with molecular weight 200 to 20,000. The moisturizing ingredients can be included in the conditioner compositions at a concentration range of 0.01 - 2.5% by weight calculated to the total composition.

The sequestering agents are selected from polycarboxy acids. The preferred one is ethylene diamine tetraacetic acid, EDTA. Typical useful concentration range for sequestering agents is of 0.01 - 2.5% by weight calculated to the total composition.

The UV filters are those oil and water soluble ones for the purpose of protecting hair colour. In other words, anionic and nonionic, oily, UV filters are suitably used in the compositions of the present invention. Suitable UV-absorbing substances are: 4-Aminobenzoic acid and the esters and salts thereof, 2-phenyl benzimidazole-5-sulfonic acid and the alkali and amine salts thereof, 4-dimethyl aminobenzoic acid and the esters and salts thereof, cinnamic acid and the esters and salts thereof, 4-methoxycinnamic acid and the esters and salts thereof, salicylic acid and the esters and salts thereof, 2,4-dihydroxybenzophenone, 2,2',4,4'-tetrahydroxy- benzophenone, 2-hydroxy-4-methoxybenzophenone and its 5-sulfonic acid or the sodium salt thereof, 2,2'-dihydroxy-4,4'-dimethoxybenzophenone, 2-hydroxy-5-chlorobenzophenone, 2,2'-dihydroxy-4-methoxybenzophenone, 2,2'-dihydroxy-4,4'-dimethoxy-5,5'-disulfobenzo-phenone or the sodium salt thereof, 2-hydroxy-4-octyloxybenzophenone, 2-hydroxy-4-methoxy-4'-methylbenzophenone, 3-benzyl-idenecampher, 3-(4'-sulfo)-benzyl-idenebornane-2-one and the salts thereof and/or 3-(4'-methyl benzylidene)-DL-campher. The amount of the UV-absorber ranges typically from 0.01 % to 2.5%, more preferably from 0.05 % to 1 % by weight, calculated to the total composition.

Natural plant extracts are incorporated usually in an amount of 0.01 % to 10 %, preferably 0.05 % to 7.5 %, in particular 0.1 % to 5 % by weight, calculated as dry residue thereof to the total composition. Suitable aqueous (e.g. steam-distilled) alcoholic or hydro-alcoholic plant extracts known per se are in particular extracts from leaves, fruits, blossoms, roots, rinds or stems of aloe, pineapple, artichoke, arnica, avocado, valerian, bamboo, henbane, birch, stinging nettle, echinacea, ivy, wild angelica, gentian, ferns, pine needles, silver weed, ginseng, broom, oat, rose hip, hamamelis, hay flowers, elderberry, hop, coltsfoot, currants, chamomile, carrots, chestnuts, clover, burr root, cocoanut, cornflower, lime blossom, lily of the valley, marine algae, balm, mistletoe, passion flower, ratanhia, marigold, rosemary, horse chestnut, pink hawthorn, sage, horsetail, yarrow, primrose, nettle, thyme, walnut, wine leaves, white hawthorn. Suitable trade products are, for example, the various "Extrapon®" products, "Herbasol^{R}", "Sedaplant^{R}" and "Hexaplant^{R}". Extracts and the preparation thereof are also described in "Hagers Handbuch der pharmazeutischen Praxis", 4^{th} Ed..

It is self-understood that the shampoos according to the invention may comprise other substances customarily used in such compositions such as preservatives, fragrances. A list of such additives can also be found in Schrader, I.c., on pp. 695 to 722.

According to the invention, as a rule only those designed as shampoo composition for hair may comprise direct acting hair dye. Suitable cationic dyestuffs are in principal those available on the market for hair colouring applications. Some examples to those are: Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green 1, Basic Red 2, Basic Red 12 Basic Red 22, Basic Red 76, Basic Violet 1, Basic Violet 2, Basic Violet 3, Basic Violet 10, Basic Violet 14 and Basic Yellow 57.

For this purpose, special reference is made to the PCT application WO 95/15144 of Ciba-Geigy AG. The cationic dyestuffs know with their CTFA adopted names Basic Yellow 87, Basic orange 31 and Basic Red 51 are especially preferred ones according to the present invention.

Cationic dyestuffs are included into the compositions of the present invention at a concentration of 0.0001 to 2%, preferably 0.0001 to 1.5% and more preferably 0.0001 to 1% by weight, calculated to total aqueous composition.

Anionic dyes may as well be used in combination with cationic direct dyes at minor quantities. The suitable ones are:
Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 6, FD&C Blue 1, Food Black 1, Food Black 2, Disperse Black 9 and Disperse Violet 1 and their alkali metal salts such as sodium, potassium.

According to the invention, anionic dyes may be included in minor quantities at a concentration around 25%, preferably not more than 10% of the total cationic dye content of the composition. It should as well be noted that anionic dyes can as well be used to color the cleansing compositions without having any hair coloring effect. In this case those should preferably be used alone and without mixing with hair direct dyes.

Additionally, the shampoo compositions of the present invention may comprise neutral dyes (HC dyes), so called nitro dyes in addition to the cationic direct dyes. Concentration of those can typically be in the range of 0.0001 to 1%, preferably 0.0001 to 0.75% and more preferably 0.0001 to 0.5% by weight calculated to total aqueous composition.

Some examples to those are: HC Blue No.2, HC Blue No.4, HC Blue No.5, HC Blue No.6, HC Blue No.7, HC Blue No.8, HC Blue No.9, HC Blue No.10, HC Blue No.11, HC Blue No.12, HC Blue No.13, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7, HC Red No.8, HC Red No.9, HC Red No.10, HC Red No.11, HC Red No.13, HC Red No.54, HC Red No.14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10, HC Yellow No.11, HC Yellow No.12, HC Yellow No.13, HC Yellow No.14, HC Yellow No.15, 2- Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol and 2-hydroxyethylpicramic acid.

The anionic, HC, neutral, dyes are always used in combination with cationic direct dyes if the compositions are designed for hair colouring purpose.

The following examples are to illustrate the invention, but not to limit. The compositions according to the invention are prepared by mixing the individual components in water, whereby it is also possible to use pre-mixtures of various ingredients.

### Example 1

| **Shampoo composition** | |
|---|---|
| Sodium lauryl ether sulfate | 11.4 (% by wt.) |
| Coco glucoside | 4.3 |
| Cocoamidopropyl betaine | 1.6 |
| Cationic polymer (Polyquaternium-1 0) | 0.3 |
| Benzylalcohol | 0.25 |
| Perfume, preservative | q.s |
| PEG-60-hydrogenated castor oil | 0.5 |
| PEG-18 Glyceryl cocoate/oleate | 1.0 |
| Glyceryl laurate | 0.2 |
| Sodium chloride | 0.3 |
| Malic acid | 0.75 |
| Lactic acid | 0.35 |
| Water | ad100.0 |

The pH of the composition is 3.5. The weight ratio of anionic/nonionic/amphoteric surfactants is 10/3.8/1.4.

As surfactants the following commercial products were used. Texapon N70 (Sodium lauryl ether sulfate), Plantacare 818 UP (Coco glucoside) and Tego Betaine F50 (Cocoamidopropyl betaine).

The volunteers were very satisfied with the skin compatability of the above shampoo composition in a monadic (meaning not comparative) home use test over a period of 6 weeks. They also evaluated the foam properties being excellent and a well as hair conditioning properties especially in terms of shine, combability, manageability and softness of hair

The above composition and the comparative compositions (see below) were tested for their skin irritation potential in an occlusive patch test using male and female volunteers backs aging 45 +- 14 years using Hayes Chamber with 25 µl volume. In detail, the shampoo compositions to be tested were diluted to a shampoo concentration of 10% by weight in tap water and homogeneously mixed.
Using a laboratory calibrated pipette 25 µl of the solutions were filled into the chambers and the samples were patched onto the backs of the volunteers. The application was repeated 3 times (the duration of skin contact in total is 72 hours). After removal of the last patch (after 72 hrs), the application areas were evaluated visually by a dermatologist. The evaluation scores were as follows:

| | |
|---|---|
| 0 | No apparent cutaneous involvement |
| 0.5 | minimal erythema |
| 1.0 | definite erythema |
| 2 | strong erythema |
| 3 | severe erythema (beet redness) |
| 4 | severe erythema with edema extending clearly beyond the area |

Such kind of tests are offered by several dermatological institutes or dermatologists having private clinics and the tests of the present invention were as well carried out in a dermatological institutes in Germany.

The comparative compositions and the control compositions were as follows:

### Comparative composition 1

The composition of the example 1 was only varied in the surfactant matter as follows

| | |
|---|---|
| Sodium lauryl ether sulfate | 14.2 (% by wt.) |
| Coco glucoside | 2.3 |
| Cocoamidopropyl betaine | 0.8 |

The rest of the composition and as well as the pH of the composition were as in example 1. The total surfactant concentration was kept constant. The surfactants are included at a weight ratio of 10/1.6/0.56 anionic/nonionic/amphoteric surfactants.

### Comparative composition 2

The composition of the example 1 was only varied in the surfactant matter as follows

| | |
|---|---|
| Sodium lauryl ether sulfate | 8.5 (% by wt.) |
| Coco glucoside | 6.5 |
| Cocoamidopropyl betaine | 2.3 |

The rest of the composition and as well as the pH of the composition were as in example 1. The total surfactant concentration was kept constant. The surfactants are included at a weight ratio of 10/7.6/2.7 anionic/nonionic/amphoteric surfactants.

The results of the occlusive multiple patch test are summarized in table I

**Table I: Results of the occlusive patch test**

| **Test composition** | **Irritation score** |
|---|---|
| Example 1 | 0.35 |
| Comparative example 1 | 0.86 |
| Comparative example 2 | 0.56 |
| Tap water | 0.04 |
| 1% SDS* solution | 1.99 |
| Commercial shampoo | 2.12 |

| | |
|---|---|
| * by weight sodium dodecyl sulfate | |

From the above it is clear that the example 1 according to the invention showed the lowest irritation score, being the mildest shampoo composition among tested. Both comparative examples showed significantly higher irritation score than the shampoo of the invention. Furthermore, the commercial shampoo which is know to contain mainly alkali salt of alkyl sulfates showed the highest irritation score, interestingly even higher than the positive control 1% by weight SDS solution. The negative control tap water showed the lowest score as expected.

## Claims

1. Hair and skin cleansing composition **characterized in that** it comprises at least one anionic surfactant, at least one nonionic surfactant and at least one amphoteric or zwitterionic surfactant at a total surfactant concentration of 1 to 50% by weight calculated to total composition and at weight ratio between 10/2.5/1 and 10/5/2 at least one hydroxy carboxylic acid and/or dicarboxylic acid selected from lactic and malic acids and having a pH below 4.5.

2. Composition according to claim 1 **characterized in that** it comprises at least one anionic surfactant, at least one nonionic surfactant and at least one amphoteric or zwitterionic surfactant at weight ratio between 10/3/1 and 10/4/2.

3. Composition according to claims 1 and 2 **characterized in that** it comprises as an anionic surfactant ethoxylated fatty alcohol sulfate.

4. Composition according to any of the preceding claims **characterized in that** it comprises as a nonionic surfactant alkyl polyglucoside.

5. Composition according to any of the preceding claims **characterized in that** it comprises as an amphoteric or zwitterionic surfactant alkylamidoalkylbetaine.

6. Composition according to any of the preceding claims **characterized in that** it has a pH between 2 to below 4.5.

7. Composition according to any of the preceding claims **characterized in that** it comprises at least one conditioning agent selected from oily substances, non ionic substances, cationic amphiphilic ingredients, cationic polymers.

8. Composition according to any of the preceding claims **characterized in that** it comprises as an acidic compound for adjusting the pH of the composition at least one hydroxycarboxylic acid and/or dicarboxylic acid at a concentration of 0.1 to 5% by weight calculated to the total composition.

9. Composition according to any of the preceding claims **characterized in that** it comprises additionally at least one direct hair dye.

10. Use of a composition according to any of the preceding claims as a mild skin and/or hair cleansing composition.

## Patentansprüche

1. Haar- und Hautreinigungsmittel, **dadurch gekennzeichnet, dass** es mindestens ein anionisches Tensid, mindestens ein nichtionisches Tensid, mindestens ein amphoterisches oder zwitterionisches Tensid in einer Gesamtmenge von 1 - 50 Gew.- %, berechnet auf die Gesamtzusammensetzung, bei einem Gewichtsverhältnis zwischen 10/2,5/1 und 10/5/2 und mindestens eine Hydroxycarboxylsäure und/oder eine Dicarboxylsäure ausgewählt aus Milch- und Apfelsäure enthält, und einen pH-Wert unter 4,5 hat.

2. Mittel nach Anspruch 1, **dadurch gekennzeichnet, dass** es mindestens ein anionisches Tensid, mindestens ein nichtionisches Tensid und mindestens ein amphoterisches oder zwitterionisches Tensid in einem Gewichtsverhältnis zwischen 10/3/1 und 10/4/2 enthält.

3. Mittel nach den Ansprüchen 1 und 2, **dadurch gekennzeichnet, dass** es als anionisches Tensid ethoxyliertes Fettalkoholsulfat enthält.

4. Mittel nach einem der vorhergehenden Ansprüche, **dadurch gekennzeichnet, dass** es als ein nichtionisches Tensid Alkylpolyglykosid enthält.

5. Mittel nach einem der vorhergehenden Ansprüche, **dadurch gekennzeichnet, dass** es als amphoterisches oder zwitterionisches Tensid Alkylamidoalkylbetain enthält.

6. Mittel nach einem der vorhergehenden Ansprüche, **dadurch gekennzeichnet, dass** es einen pH-Wert zwischen 2 bis unter 4,5 hat.

7. Mittel nach einem der vorhergehenden Ansprüche, **dadurch gekennzeichnet, dass** es mindestens ein Konditionierungsmittel, ausgewählt aus öligen Substanzen, nichtionischen Substanzen, kationisch amphiphilischen Bestandteilen und kationischen Polymeren enthält.

8. Mittel nach einem der vorhergehenden Ansprüche, **dadurch gekennzeichnet, dass** es als eine saure Komponente zum Einstellen des pH-Wertes des Mittels mindestens eine Hydroxycarbonsäure und/oder Dihydroxycarbonsäure in einer Menge von 0,1 bis 5 Gew.- %, berechnet auf die Gesamtzusammensetzung, enthält.

9. Mittel nach einem der vorhergehenden Ansprüche, **dadurch gekennzeichnet, dass** es zusätzlich mindestens einen direktziehenden Haarfarbstoff enthält.

10. Verwendung eines Mittels nach einem der vorhergehenden Ansprüche als ein mildes, Haut- und/oder Haarwaschmittel.

## Revendications

1. Composition de nettoyage pour les cheveux et la peau **caractérisée en ce qu'**elle comprend au moins un tensioactif anionique, au moins un tensioactif non ionique, au moins un tensioactif amphotère ou zwitterionique à une concentration totale de tensioactifs de 1 à 50 % en poids calculée per rapport à la composition totale et dans un rapport pondéral entre 10/2,5/1 et 10/5/2 et au moins un acide hydrocarboxylique et/ou un acide dicarboxylique choisi parmi les acides lactique et malique et ayant un pH inférieur à 4,5.

2. Composition selon la revendication 1 **caractérisée en ce qu'**elle comprend au moins un tensioactif anionique, au moins un tensioactif non ionique et au moins un tensioactif amphotère ou zwitterionique dans un rapport pondéral entre 10/3/1 et 10/4/2.

3. Composition selon les revendications 1 et 2 **caractérisée en ce qu'**elle comprend comme tensioactif anionique un sulfate d'alcool gras éthoxylé.

4. Composition selon l'une quelconque des revendications précédentes **caractérisée en ce qu'**elle comprend comme tensioactif non ionique un polyglucoside d'alkyle.

5. Composition selon l'une quelconque des revendications précédentes **caractérisée en ce qu'**elle comprend comme tensioactif amphotère ou zwitterionique une alkylamidoalkylbétaine.

6. Composition selon l'une quelconque des revendications précédentes **caractérisée en ce qu'**elle a un pH entre 2 et moins de 4,5.

7. Composition selon l'une quelconque des revendications précédentes **caractérisée en ce qu'**elle comprend au moins un agent de conditionnement choisi parmi les substances huileuses, les substances non ioniques, les ingrédients amphiphiles cationiques et les polymères cationique.

8. Composition selon l'une quelconque des revendications précédentes **caractérisée en ce qu'**elle comprend comme composé acide pour ajuster le pH de la composition au moins un acide hydroxycarboxylique et/ou un acide dicarboxylique à une concentration de 0,1 à 5 % en poids calculée par rapport à la composition totale.

9. Composition selon l'une quelconque des revendications précédentes **caractérisée en ce qu'**elle comprend en outre au moins un colorant capillaire direct.

10. Utilisation d'une composition selon l'une quelconque des revendications précédentes comme composition de nettoyage douce pour la peau et/ou les cheveux.
